# EUROPÄISCHE PATENTANMELDUNG

(11) **EP 1 238 685 A1**
(43) Veröffentlichungstag der Anmeldung: **11.09.2002**
(21) Anmeldenummer: 01112942.6
(22) Anmeldetag: 06.06.2001
(51) Int. Cl.: A61N 5/10, A61B 6/04

(54) **Patientenpositionierungssystem für die Radiotherapie**

(30) Priorität: 05.03.2001 EP 01104553
(71) Anmelder: BrainLAB AG, 85551 Kirchheim/Heimstetten (DE)
(72) Erfinder: Erbel, Stephan, 81677 München (DE); Fröhlich, Stephan, 85609 Aschheim (DE)
(74) Vertreter: Schwabe - Sandmair - Marx

(57) **Zusammenfassung**

Die Erfindung betrifft ein Patientenpositionierungssystem für die Radiotherapie mit einer Vorrichtung zur Patientenpositionierung im Rahmen der Behandlungsplanung mit Bilderfassung und/oder der Durchführung einer Bestrahlungsbehandlung, mit einer Patientenliege (3, 42, 50), auf der ein Patient gelagert wird, bei der Mittel vorgesehen sind, mit denen dieselbe Patientenliege (3, 42, 50) sowohl an einer Planungscouch (8) bei der Bilderfassung im Rahmen der Behandlungsplanung als auch an einem Bestrahlungstisch (1, 44) angebracht werden kann, und dadurch, dass an der Patientenliege ein Referenzmittel (54) vorgesehen ist, mit dem die Lage des immobilisierten Patienten bzw. eines markierten Bestrahlungsziels gegenüber der Patientenliege (3, 42, 50) definiert bestimmbar ist. In diesem Rahmen wird auch ein entsprechendes Verfahren bereitgestellt, sowie eine Transport- und Liegen-Verstellvorrichtung.

## Beschreibung

Die vorliegende Erfindung betrifft ein Patientenpositionierungssystem für die Radiotherapie bzw. für die Bestrahlungstherapie oder Radiochirurgie. Grundsätzlich wird im Rahmen solcher Bestrahlungsbehandlungen so vorgegangen, dass zunächst einmal die Bestrahlungsplanung erfolgt, wobei von dem Patienten oder von dem Bereich des Patienten um das Bestrahlungsziel herum Bildaufnahmen gemacht werden, beispielsweise Computertomographieaufnahmen. Über bekannte Markierungssysteme wird die Position des Behandlungsziels registriert. Danach wird gemäß heutigem Standard der Patient von der Liege am bildgebenden Gerät herabgeholt und geht selbständig zum Bestrahlungsraum um sich dort auf eine weitere Liege am Bestrahlungsgerät zu legen. Dort wird über ein Trackingsystem versucht, über die Lage der am Patienten angebrachten Markierungen die aktuelle Lage des Bestrahlungsziels zu bestimmen bzw. den Patienten mittels eines Trackingsystems so zu positionieren, dass das Behandlungsziel im Isozentrum des Bestrahlungsgerätes liegt. Typischerweise findet die Aufnahme der Bilddaten für die Planung nicht am gleichen Tag wie die Behandlung statt.

Einen großen Nachteil bringt dieses Vorgehen schon wegen der Bewegung des Patienten zwischen den beiden Patientenliegen mit sich. Markierungen, die meist auf der Haut des Patienten angebracht sind, verschieben sich bei dieser Umlagerung zueinander und im Verhältnis zum Bestrahlungszielpunkt. Dadurch wird die Positionierung bei der Bestrahlung am Bestrahlungsgerät ungenau und somit auch die Bestrahlung selbst, was den Erfolg der Behandlung möglicherweise zumindest teilweise in Frage stellt.

Ein ähnliches Problem tritt dann auf, wenn die Position des Bestrahlungsziels vom jeweiligen Atmungszustand des Patienten abhängt. Wurde der Planungsdatensatz des Patienten zum Beispiel bei angehaltener Atmung aufgenommen, so stellt dieser Datensatz den Patienten und die Position des Zielvolumens bei einer bestimmten Lungenfüllung dar. Um diese Daten auf den Zustand des Patienten zum Zeitpunkt der Bestrahlung übertragen zu können, müssten die Lungenfüllungen zu beiden Zeitpunkten genau übereinstimmen. Kann der Patient während der Bestrahlung frei atmen, so ist dies höchstens zu zwei Zeitpunkten pro Atemzug der Fall. Außerdem wird die Atmungsdrift, also eine Verschiebung des Lungengrundvolumens über mittlere Zeiträume, ein Problem darstellen.

Auch hier tritt das Problem der Übertragbarkeit des 3D-Planungsdatensatzes auf den Zustand des Patienten zum Zeitpunkt der Bestrahlung auf.

Bisherige Lösungsansätze für das oben zuletzt genannte Problem machten sich zunutze, dass die Atmung eines Patienten durch das Beobachten der äußeren Kontur des Brustkorbes und des Bauches verfolgt werden kann. Werden auf der Haut des Patienten Markierungen angebracht, so können diese mit der Position von inneren Zielvolumina korreliert werden. Wird jedoch der Patient nach dem Durchführen der bildgebenden Untersuchung (zum Beispiel CT, MR, SPECT, PET) umgelagert, wobei der Patient typischerweise aufstehen, in den Bestrahlungsraum gehen und sich dort auf eine andere Liege legen muss, so verrutschen auch hier wieder die Hautfettschichten des Patienten und damit auch die darauf angebrachten Markierungen. Daraus resultiert wiederum zum einen eine ungenaue Position des Patientenkoordinatensystems als auch eine deutliche Verfälschung einer eventuell zuvor ermittelten Korrelation zwischen der atmungsabhängigen Position des Zielvolumens und den externen Markierungen. Aus diesem Grund wird gemäß einem bekannten Lösungsansatz die Korrelation direkt auf dem Tisch des Bestrahlungsgerätes vorgenommen. Dies ist wiederum deshalb unvorteilhaft, weil die nutzbaren bildgebenden Verfahren entweder von minderwertiger Qualität verglichen mit den zuvor beschriebenen Geräten sind, oder aber eine hohe Investition erfordern, da entweder das Therapiegerät oder das bildgebende Gerät genutzt werden kann, nicht jedoch beide gleichzeitig.

Die Erfindung hat es sich zur Aufgabe gemacht, die oben aufgeführten Probleme zu lösen. Diese Lösung erfolgt erfindungsgemäß durch eine Vorrichtung gemäß dem beiliegenden Anspruch 1 sowie ein Verfahren gemäß dem Anspruch 20, und basiert einerseits darauf, dass dieselbe Patientenliege zur Lagerung des Patienten am bildgebenden Gerät und bei der Bestrahlung verwendet wird. Mit anderen Worten kann der Patient auf der einen Liege gelagert bleiben und wird mit dieser vom Planungssystem zum Bestrahlungsgerät verbracht. Dadurch verringern sich bei einem immobilisierten Patienten schon sehr stark diejenigen Verschiebungen, die aus der Umlagerung resultieren. Ferner basiert die vorteilhafte Genauigkeit des erfindungsgemäßen Systems noch darauf, dass an der transportablen Patientenliege ein Referenzmittel vorgesehen ist, mit dem die Lage des immobilisierten Patienten bzw. eines oder mehrerer markierter Referenzpunkte an der Körperoberfläche des Patienten gegenüber einem fest mit der Patientenliege verbundenen Punkt feststellbar ist. Mit diesem Referenzmittel hat man noch einen zusätzlichen festen Bezugspunkt, der in der Nähe des durch die Markierungen am Patienten bestimmten Zielvolumens liegt und der als feste Positionierungsgröße sehr hilfreich ist, und zwar insbesondere dann, wenn ein Bestrahlungszielvolumen seine Position mit der Atmung des Patienten ändert. Bei den bisherigen Verfahren, bei denen lediglich die Position der Patientenmarkierungen zur atemabhängigen Bestrahlung verwendet wurde, fehlte nämlich eine externe Referenz, um zum Beispiel eine Drift, d. h. eine Verschiebung des Lungen-Grundvolumens zu erkennen. Zusätzlich zu dem oben schon angesprochenen labilen Verhältnis zwischen Patientenmarkern und Zielvolumen konnte dies Ungenauigkeiten bei der Bestrahlung mit sich bringen. Durch das Fehlen einer externen Referenz, welche es erlaubt einen absoluten Lungenfüllungskoeffizienten zu bestimmen, ist es demnach bisher nicht möglich gewesen getriggerte Bildaufnahmen einem bestimmten Atemzustand während der Behandlung zuzuordnen.

Die Tatsache, dass gemäß der Erfindung nunmehr ein solches Referenzmittel an der Patientenliege bereitgestellt wird, ermöglicht aber gerade die Herstellung sozusagen "absoluter" Patientenmarkerpositionen, und zwar gegenüber dem fest an der Liege angeordneten Referenzmittel. Dadurch lassen sich jedwede Verschiebungen der Patientenmarkierungen untereinander, aber auch gegenüber dem System der Patientenliege erkennen und korrigieren bzw. berücksichtigen, ob sie nun entstanden sind, weil sich der Patient bewegt hat, oder ob sie aus der Atmungsbewegung herrühren. Durch relativ zu einem absoluten Lungenfüllungswert getriggerte Bildaufnahmen werden im Behandlungsverlauf getriggerte Bestrahlungen mit wesentlich höherer Präzision möglich werden, d. h. es wird immer nur dann bestrahlt, wenn sich das Zielvolumen auch an einem bestimmten Ort befindet. In diesem Sinne verhilft die vorliegende Erfindung zur Vermeidung von Positionierungsfehlern und einer genauen Berücksichtigung von atmungsbedingten Organverschiebungen, und damit auch zu sehr viel genaueren und wirksameren Bestrahlungen als dies derzeit gemäß dem Stand der Technik möglich ist.

Bei einer bevorzugten Ausführungsform der erfindungsgemäßen Vorrichtung weist diese, wie oben schon angedeutet, ein Trackingsystem auf, welches sowohl die Positionen von auf dem Patienten angebrachten Markierungen als auch die des Referenzmittels, insbesondere eines mit einer Markeranordnung bestückten Referenzsternes an der Liege erfassen kann. Hierzu ist insbesondere noch anzuführen, dass ein solcher Referenzstern, der beispielsweise eine Anordnung von drei Infrarot-reflektierenden Markern aufweisen kann, durchaus zusammen mit den Patientenmarkierungen während der Schichtbildaufnahme (CT) innerhalb des Tomographenraumes beobachtet werden kann, so dass getriggerte Aufnahmen möglich sind, die später eine getriggerte Bestrahlung dann erlauben, wenn die Patientenmarker und die Marker auf dem Referenzstern in einem definierten Verhältnis stehen, und zwar +/- einem gestatteten Toleranzbereich um ihre Lage (relativ zum Referenzmittel) während der Aufnahme der Schichtbilder. Als Erweiterung ist hier noch denkbar, bei mehreren definierten Zuständen (Abstandsverhältnissen) von Patientenmarkern und Referenzmarkern an der Liege verschiedene Planungs-Bildaufnahmesätze des gleichen Gebietes aufzunehmen und damit die Bewegung des Zielpunktes zu analysieren. Der "Beam-On-Range", d. h. der Zeitraum, über den der Behandlungsstrahl bei der Behandlung angeschaltet ist, kann dann individuell für eine vorbestimmte obige Markerbeziehung definiert werden. Man erhält also schon bei der Bildaufnahme, d. h. bei der Planung einen ganzen Satz von Anfangspunkten.

Um das erfundene System implementieren zu können ist es jedoch unter Umständen unvorteilhaft, nur konventionelle, dem Stand der Technik entsprechende Geräte einzusetzen. Aus diesem Grund beinhaltet die vorliegende Erfindung eine neuartige Patientenliege, deren Einsatz ihrerseits nur im Zusammenspiel mit einer neuartigen Winkelverstelleinrichtung zur Positionierung des Patienten während der Bestrahlung möglich wird.

Vorteilhafterweise umfasst die erfindungsgemäße Vorrichtung eines oder mehrere der folgenden Immobilisierungsmittel zur Immobilisierung des Patienten auf der Patientenliege:
- Vakuumfolien mit entsprechenden Unterdruckpumpen
- Elastische Bänder oder Folien, die über den Patienten gespannt werden
- Speziell an die Form des Patienten angepasste Vakuumkissen
- Speziell an die Form des Patienten angepasste thermoplastische Körpermasken
- Formkissen oder -blöcke, die an die Form des Patienten angepasst werden.

Eine erfindungsgemäße Vorrichtung zur Patientenpositionierung im Rahmen der Behandlungsplanung mit Bilderfassung und/oder der Durchführung einer Bestrahlungsbehandlung, mit einer Patientenliege, auf der ein Patient gelagert wird, insbesondere eine Vorrichtung, wie sie oben beschrieben wurde, kann eine Verstelleinrichtung aufweisen, mit der die Patientenliege um mindestens zwei Achsen gedreht werden kann und die zwischen der Patientenliege und einer Stützeinrichtung für die Patientenliege, insbesondere einer Planungscouch bzw. einem Behandlungstisch angeordnet ist. Hierdurch wird das Problem angegangen, dass wohl in den meisten Fällen bei der Bilderfassung und bei der Bestrahlung der Rotationswinkel um die Körperlängsachse und die horizontale Querachse hierzu nicht identisch ist. Deshalb ist der Weg, den der Strahl durchschreitet, bis er zum Behandlungsziel kommt meist nicht identisch mit dem Weg, der bei der Bestrahlungsplanung im Sinne einer optimalen Behandlung festgelegt wurde. Stereotaktische Radiochirurgie setzt die Möglichkeit voraus, ein bestimmtes Zielgebiet im Körper des Patienten aus einer bestimmten Richtung bestrahlen zu können. Durch die begrenzte Steifigkeit der Tische von Linearbeschleunigern und ähnlichen Radiochirurgieund Radiotherapiesystemen kommt es jedoch oft dazu, dass das zu behandelnde Körperteil oder der gesamte Patient nach der Fixierung auf dem Behandlungsgerät eine Winkelverdrehung erfährt, die entsprechen korrigiert werden muss. Um diese Korrektur durchzuführen, werden beim Stand der Technik Systeme verwendet, bei denen der Kopf des Patienten innerhalb eines bestimmten Winkelbereiches um die Quer- und Längsachse gedreht werden kann.

Ein solches Patiententisch- bzw. Patientenliegesystem mit einer Verstelleinrichtung gemäß dem Stand der Technik ist in schematischer Darstellung in Figur 10 gezeigt. Die Verstelleinrichtung C wird auf der Behandlungsliege B des Therapiegerätes befestigt und nimmt den Kopf mittels eines Masken- oder Schraubringsystems D auf. Um die nötige Steifigkeit zu erreichen, sind diese Verstellmechanismen schwer und relativ groß. Um die Kräfte und Momente aus ihrem eigenen Gewicht und dem Gewicht des Patientenkopfes aufzunehmen und auf die Behandlungsliege übertragen zu können, ist es dabei notwendig, relativ solide und schwere Aufnahmevorrichtungen am Patiententisch vorzusehen. Diese Aufnahmevorrichtungen sind jedoch insofern problematisch, als sie nicht so strahlungsdurchlässig sein können wie eine einfache, durchgehende (Karbon-)Liege. Ein zusätzliches Problem ist die Größe der Vorrichtungen, die das Kollisionsrisiko zwischen Liege und dem bewegten Teil des Bestrahlungsgerätes deutlich erhöht, und den Transport der Liege wesentlich erschwert. Außerdem kann die Winkelverstellung nicht für extrakranielle Bestrahlungen genutzt werden, und gerade dieser Mangel ist durch die in letzter Zeit erzielten Fortschritte auf dem Gebiet der extrakraniellen Radiotherapie ein wesentlicher Nachteil.

Die oben beschriebene Vorrichtung löst dieses Problem durch die Anordnung der Verstelleinrichtung zwischen der Patientenliege und der Stützeinrichtung für die Patientenliege, d. h. zum Beispiel der CT-couch bzw. des Behandlungstisches. Dadurch kann die Winkelverstellung für alle Patientenkörperteile erfolgen und die Verstelleinrichtung ist nicht mehr im Weg bei der Aufnahme und stört nicht mehr am Bestrahlungsgerät.

Eine Kombination mit einem Erfassungssystem (Trackingsystem) ist möglich.

Die Verstelleinrichtung kann ein unabhängiges Drehen der Patientenliege um mindestens eine Achse quer und eine Achse längs zur Liege ermöglichen. Sie besteht vorzugsweise aus einer Grund- und einer Deckplatte, die über eine Dreipunkt- oder Vierpunktlagerung miteinander beweglich verbunden sind. Hierbei ist es möglich, die Verstelleinrichtung so auszugestalten, dass sie mindestens zwei Auflager aufweist, die als höhenverstellbare, pneumatische, hydraulische, piezoelektrische oder elektromechanische Auflager, direkt oder mittels eines Hebelsystems verstellbar, ausgebildet sind.

Die Verstelleinrichtung weist bevorzugt eine vordere, kopfseitige Auflagerung und eine hinter, fußseitige Auflagerung auf, wobei mindestens eine der Auflagerungen als Kugelgelenkoder Kardanlage ausgebildet ist. Die vordere oder hintere Auflagerung kann dabei Kniehebel mit beidseitigen Kugelgelenken aufweisen.

Bei einer Ausgestaltung der erfindungsgemäßen Vorrichtung umfasst die Lagerung der Deckplatte auf der Grundplatte ein Wellen-Nut-Gelenk mit zwei translatorischen und zwei rotatorischen Freiheitsgraden, welches ein seitliches Verschieben der Deckplatte gegenüber der Grundplatte verhindert. Einerseits kann die Vorrichtung Verstellmittel aufweisen, welche die Rotation der Liege um ihre Längsachse durch gegenläufiges Verstellen von zwei kopf- oder fußseitigen Auflagern bewirken, andererseits können die Verstellmittel auch die Rotation der Liege um ihre Querachse durch gleichläufiges Verstellen eines oder zweier kopf- oder fußseitiger Auflager bewirken. Natürlich kann beides realisiert werden. Die Verstellmittel können ebenfalls die Rotation der Liege um ihre Querachse durch gleichzeitiges Verstellen mindestens eines kopfseitigen und mindestens eines fußseitigen Auflagers bewirken.

Insbesondere bei der Verwendung von Kniehebeln muss die Verstell-Geschwindigkeit beachtet werden. Deshalb weist die Vorrichtung bei einer Ausführungsform Verstellmittel auf, welche die Rotation der Liege um ihre Querachse durch gezieltes Einstellen der Verstell-Geschwindigkeit mindestens eines kopfseitigen und mindestens eines fußseitigen Auflagers bewirken, wobei die Lage der scheinbaren Drehachse in eine gewollte Position gebracht werden kann. Ferner kann sie Verstellmittel aufweisen, welche die Rotation der Liege um ihre Längsachse durch gezieltes Einstellen der Verstell-Geschwindigkeit mindestens zweier Auflager bewirkt, wodurch die Lage der scheinbaren Drehachse in eine gewollte Position gebracht werden kann.

Erfindungsgemäß wird ferner eine Vorrichtung zur Patientenpositionierung im Rahmen der Behandlungsplanung mit Bilderfassung und/oder der Durchführung einer Bestrahlungsplanung bereitgestellt, mit einer Patientenliege, auf der ein Patient gelagert wird, insbesondere eine Vorrichtung, wie sie oben beschrieben wurde, wobei diese Vorrichtung eine Transportvorrichtung, insbesondere einen Transportwagen zum Transportieren der Liege und des darauf liegenden Patienten umfasst. Eine solche Transportvorrichtung kann in optimaler Weise die schon vorher beschriebenen vorteilhaften Ausführungsformen der Patientenlagerung ergänzen, und bei einer bevorzugten Ausgestaltung sind dabei zur Verbindung von Patientenliege und Transportwagen zwei Einklinkmechanismen vorgesehen, zum Einhängen der Patientenliegen an einem Tisch eines Behandlungssystems und an einer Couch eines bildgebenden Planungssystems. Das Ein- und Aushängen der Liege beim Tisch des Bestrahlungsgerätes oder bei der Couch des Planungssystems kann einerseits über die Höhenverstellung des Tisches bzw. der Couch erfolgen; andererseits besteht auch die Möglichkeit, dies über die Höhenverstellung der Transportvorrichtung zu realisieren.

Ferner stellt die Erfindung ein Verfahren zur Behandlungsplanung mit Bilderfassung und/oder Durchführung einer Bestrahlungsbehandlung zur Verfügung. Bei diesem Verfahren wird der Patient auf einer Patientenliege gelagert, dieselbe Patientenliege wird sowohl an einer Planungscouch bei der Bilderfassung im Rahmen der Behandlungsplanung als auch an einem Bestrahlungstisch genutzt, und die Lage des immobilisierten Patienten bzw. eines markierten Bestrahlungsziels wird gegenüber der Patientenliege über ein Referenzmittel bestimmt, das fest an der Patientenliege vorgesehen ist. Natürlich hat das erfindungsgemäße Verfahren auch die schon anhand der Vorrichtung vorher beschriebenen Vorteile gegenüber dem Stand der Technik.

Insbesondere wird bei einem Verfahren gemäß der vorliegenden Erfindung die Liege, auf welcher der Patient liegt, von der Planungscouch abgenommen, zum Bestrahlungsgerät transportiert und dort wieder befestigt. Es kann ein Trackingsystem am bilderfassenden Gerät sowohl die Position von auf dem Patienten angebrachten Markierungen als auch die der Referenzmarkierungen auf der Liege erfassen bzw. nach diesen eingestellt werden. Der Patient ist bevorzugt so auf der Liege zu fixieren, dass er sich weder relativ zur Liege verschieben kann, noch die Lage seiner Körperteile zueinander ändern kann.

Wird das erfindungsgemäße Verfahren zur atemsynchronisierten Bestrahlung verwendet, wird der fixierte Patient mit externen Markierungen bestückt, wobei anhand der Position dieser Markierungen relativ zu dem Referenzmittels verifiziert wird, dass der Patient die Atmung während der Aufnahme der Planungsdaten angehalten hat.

Gemäß einer weiteren Ausgestaltung des erfindungsgemäßen Verfahrens wird die relative Position von Patientenmarkierungen zu dem Referenzmittel ermittelt, und bei Übereinstimmung mit toleranzbehafteten Vorgaben wird eine Bildaufnahme gestartet oder gestoppt, wenn der Patient die Luft anhält, die Atmung wieder aufnimmt, eine bestimmte Lungenfüllung erreicht hat oder eine bestimmte Lungenfüllung in einer vorgegebenen Atemphase (zum Beispiel währen des Einatmens oder des Ausatmens) durchschreitet. Es ist ferner möglich, den Patienten nur zu Zeitpunkten zu bestrahlen, in denen die relative Position von Patientenmarkierungen zur Position des fest auf der Liege angebrachten Referenzmittels in einem definierten Toleranzbereich um die relative Position liegt, die während der Aufnahme eines Planungsdatensatzes vorlag (getriggerte Bestrahlung).

Die Erfindung wird im Weiteren anhand bevorzugter Ausführungsformen näher erläutert. In den beiliegenden Zeichnungen zeigen:
- Figur 1: ein Patiententisch-Patientenliege-System mit Verstelleinrichtung gemäß der vorliegenden Erfindung in einer Schrägansicht bei einem Bilderfassungssystem und in einer Seitenansicht, wie es zur Bestrahlung verwendet wird;
- Figur 2: eine Verstelleinrichtung gemäß der Erfindung in oberer und seitlicher Schnittansicht;
- Figur 3: ein hinteres Lager der Verstelleinrichtung in seitlicher, vergrößerter Ansicht;
- Figur 4: eine Frontalansicht einer Ausführungsvariante der Verstelleinrichtung mit zwei Kniehebeln und einem zusätzlichen Führungsgelenk;
- Figur 5: Schnitt-Seitenansichten, die eine Verstellmöglichkeit für die Kniehebel der Verstelleinrichtung illustrieren;
- Figur 6: eine Frontalansicht der Verstelleinrichtung bei gegenläufigem Verstellen zweier Kniehebel;
- Figur 7: eine Ausführungsvariante einer selbsttragenden Karbonliege auf der Couch eines konventionellen Linearbeschleunigers;
- Figur 8: eine Ausführungsform eines Transportsystems für eine Patientenliege;
- Figur 9: eine Ausführungsform eines Mechanismus zum Ein- und Ausklinken der Liege auf der Verstelleinrichtung oder direkt auf der Couch; und
- Figur 10: ein Patiententisch-Patientenliege-System mit Verstelleinrichtung gemäß dem Stand der Technik in schematischer Darstellung.

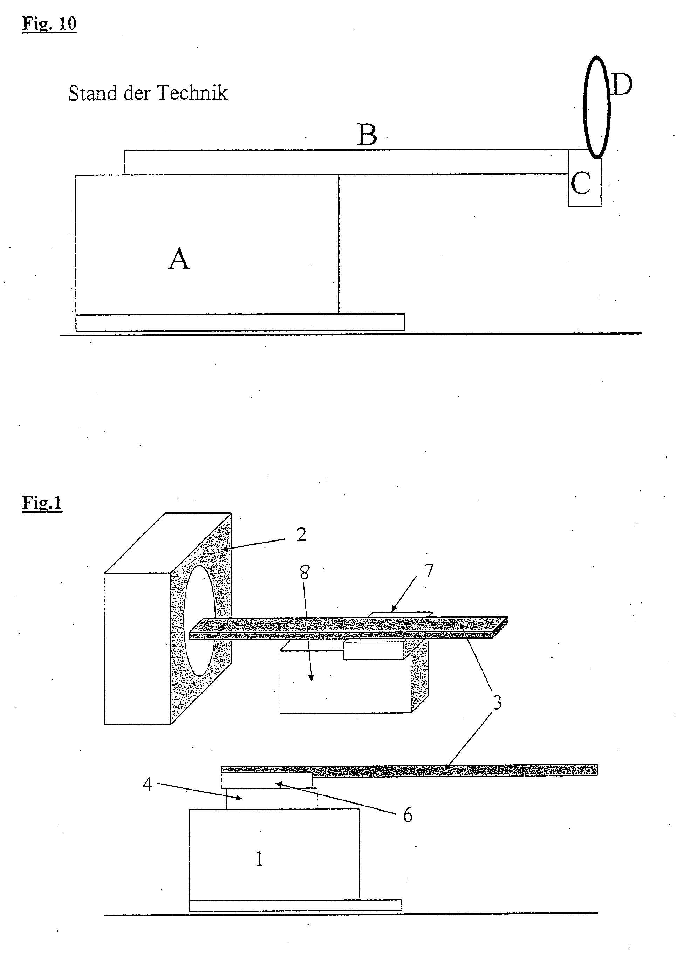

Die Figur 1 zeigt ein Patiententisch-Patientenliege-System mit Verstelleinrichtung gemäß der vorliegenden Erfindung in einer Schrägansicht bei einem Bilderfassungssystem (Planung) und einer Seitenansicht, wie es beispielsweise bei einer Bestrahlungstherapie unter einem LINAC (LINear ACcellerator) verwendet wird. Dargestellt sind in der oberen Schrägansicht (beim Planungsschritt): ein Planungssystem bestehend aus einer Patientencouch 8 und einem bildgebenden Gerät 2, beispielsweise einem CT-Gerät; und ein schematisch dargestellter Klinkmechanismus 7, mit dem eine Patientenliege 3 an der Couch 8 befestigt werden kann. In der unteren Darstellung ist die Liege 3 zur Bestrahlung auf einem Patiententisch 1 aufgebracht und eine Verstelleinrichtung 6 ist zwischen einem weiteren Klinkmechanismus 4 am Patiententisch 1 und der Patientenliege 3 eingesetzt.

Anstatt der bisher üblichen Anordnungsreihenfolge der Verbindungsstruktur: Tisch (A)-Liege (B)-Verstellmechanismus (C) (siehe Figur 10) wird die Verbindungsstruktur: Tisch (1, 8) - Verstellmechanismus (6)- Liege (3) implementiert. Dadurch lässt sich demnach die Winkelkorrektur auch für Behandlungen außerhalb des Kopfbereiches nutzen. Der zweite Vorteil dieser Vorgehensweise ist, dass die Liege 3, auf der der Patient liegt, auch im Halsbereich durchgehend aus gut strahlungsdurchlässigem Karbonmaterial gestaltet werden kann; Verstärkungen, Auflager- und Verschraubungspunkte auf der Patientenliege 3 entfallen vollständig.

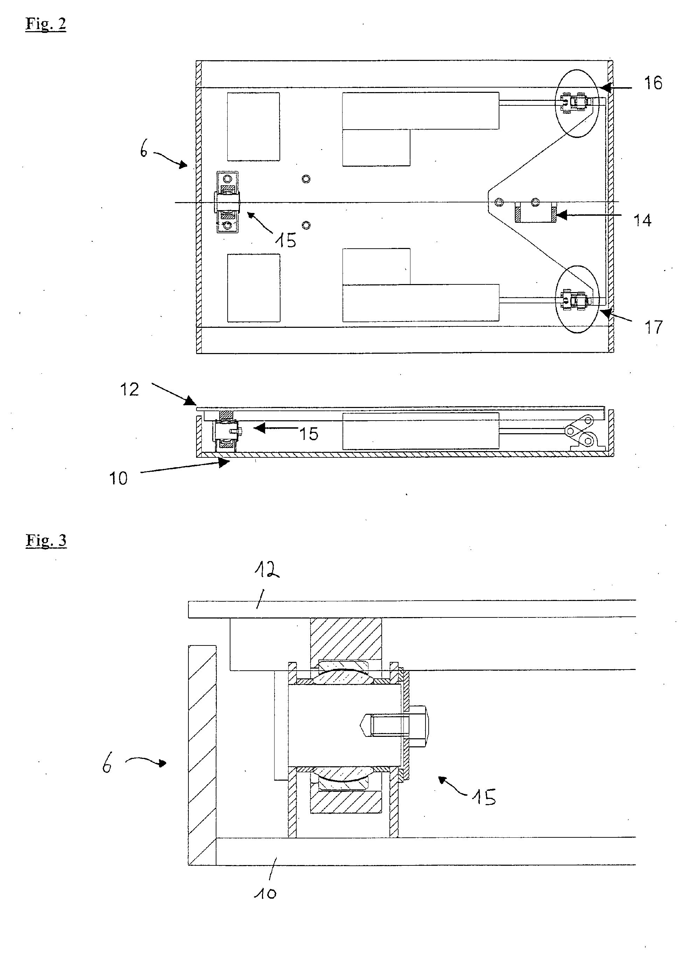

Da das hier dargestellte System auch dazu gedacht ist, bereits existierende Bestrahlungs-Systeme aufzurüsten, ist es besonders wichtig, dass die Verstelleinrichtung 6 in einen stark eingeschränkten Bauraum integriert werden kann. Diese Anforderung ist jedoch mit konventionellen Systemen mit zwei Drehachsen nicht realisierbar. Das erfundene System nutzt deshalb eine spezielle Aufhängung in der Verstelleinrichtung, wobei eine bevorzugte Ausführung im Folgenden anhand der Figur 2 beschrieben wird.

Die Figur 2 zeigt eine Verstelleinrichtung 6 gemäß der Erfindung in oberer und seitlicher Schnittansicht. Sie weist eine Grundplatte 10 und eine Deckplatte 12, auf. Die Verbindung zwischen der Grundplatte 10 auf dem Tisch des Behandlungsgerätes und der Deckplatte 12 auf der die (Karbon-) Liege 3 befestigt wird, ist über eine Dreipunktlagerung 15, 16, 17, die durch ein zusätzliches Seitenführungsgelenk 14 ergänzt wird, realisiert. Das hintere Lager 15 (etwa auf Höhe der Füße des Patienten) ist ein Kugel-Gelenk-Lager mit drei rotatorischen und keinem translatorischen Freiheitsgrad. Es ist in Figur 3 nochmals vergrößert gezeigt. Alternativ kann dieses Lager auch als Kardangelenk ausgeführt werden.

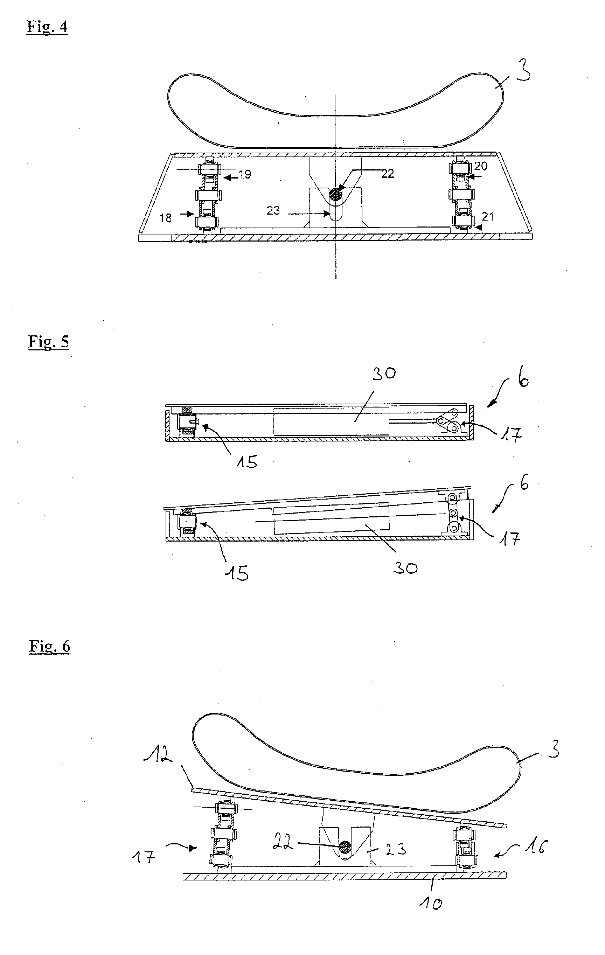

Die vorderen zwei Auflager 16, 17 bestehen aus Kniehebeln, die an der Grund- und Deckplatte 10, 12 jeweils mit Kugelgelenken 18, 19, 20, 21 fixiert sind, die in der Frontalansicht der Figur 4 am besten zu sehen sind.

Das Seitenführungsgelenk 14 befindet sich in der Nähe der Mittelgeraden zwischen beiden Kniehebeln, und weist zwei rotatorische und zwei translatorische Freiheitsgrade auf. Eine bevorzugte Ausführung dieses Gelenks ist eine Welle 22, die in einem Spalt 23 geführt wird. Alternativ kann dieses Gelenk auch entfallen, wenn an der anderen Seite des Mechanismus ein Lager mit nur zwei rotatorischen Freiheitsgraden gewählt wird.

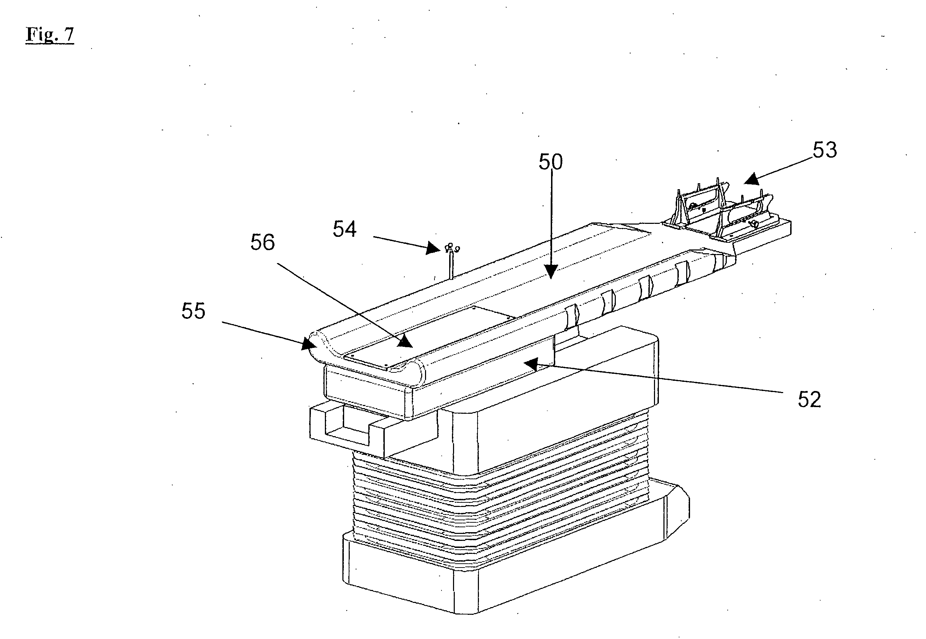

In Figur 5 sind Schnitt-Seitenansichten dargestellt, die eine Verstellmöglichkeit für die Kniehebel 16, 17 der Verstelleinrichtung 6 illustrieren, und die Figur 6 zeigt eine Frontalansicht der Verstelleinrichtung 6 bei gegenläufigem Verstellen zweier Kniehebel 16, 17. Die zwei Knichebel 16, 17 können über Elektrozylinder (30) (je ein Spindel-Mutter-System), hydraulische Zylinder oder pneumatische Zylinder angesteuert werden.

Werden die beiden Kniehebel 16, 17 in die gleiche Richtung gespreizt, so lässt sich die Liege 3 um eine Achse quer zum Patienten drehen, werden die Kniehebel 16, 17 in unterschiedliche Richtungen gespreizt, so lässt sich ein Drehen um eine Achse die in etwa parallel zur Längsachse des Patienten ist realisieren.

Um ein Drehen um die Hauptachsen (Achse durch das hintere Kugelgelenk 15, senkrecht zur Symmetrieebene des Patienten, und Achse parallel zur Symmetrieebene des Patienten) zu realisieren, ist es notwendig die effektive Höhe der Kniehebel 16, 17 mit gleicher beziehungsweise gegenläufiger Geschwindigkeit zu verändern. Eine solche gegenläufige Verstellung führt beispielsweise zu einem Zustand, wie er in der Figur 6 dargestellt ist. Da die Kniehebel 16, 17 eine vom Einknickwinkel abhangige Weg- und Kraftübersetzung aufweisen, ist es dazu nötig die Kniehebel mit Geschwindigkeiten unterschiedlichen Betrags, die in Abhängigkeit vom Knickwinkel beider Kniehebel errechnet werden, anzusteuern. Ist dies realisiert, so ist es möglich die Liege 3 um die Längsachse zu drehen, ohne dass dadurch der Winkel um die Querachse oder die Höhe der Liege 3 verändert wird.

Alternativ kann das Kugelgelenk 15 auch durch einen dritten Kniehebel mit einseitigem Kugelgelenk und einem einfachen Drehlager auf der anderen Seite dieses Kniehebels ausgeführt werden (nicht dargestellt). Bei einer solchen Ausführungsform befindet sich anstelle des Kugelgelenklagers der dritte Kniehebel, der ebenfalls über Kugelgelenklager an der Grund- und Deckplatte befestigt wird. Mit Hilfe dieses dritten Hebels ist es zusätzlich möglich, den Patienten um eine virtuelle Querachse zu drehen, deren Lage durch das Verhältnis der effektiven Geschwindigkeiten des einzelnen Kniehebels bezogen auf die effektive Geschwindigkeit der beiden Kniehebel auf der anderen Seite eingestellt werden kann. Auf diese Weise ist es möglich den auf der Liege liegenden Patienten um einen bestimmten Punkt zu drehen, vorzugsweise um den Punkt der behandelt werden soll. Im Falle eines Linearbeschleunigers ist dies das Isozentrum.

Die Figur 7 zeigt eine mögliche Ausführungsform einer Patientenliege 50 auf der Verstelleinrichtung 6. Ein Kopfhalter 53 ist gezeigt, und dieser kann nicht verdreht und deswegen aus sehr dünnem und dadurch strahlungsdurchlässigem Karbon realisiert werden. Bei 55 kann eine Endplatte aus Metall vorgesehen werden, die dazu dienen kann, die Liege 50 an einer Verstelleinrichtung 52 zu befestigen, oder auch dazu genutzt werden kann, Fußhalter oder ähnliche Zusatzgeräte mit der Liege 50 zu verbinden. Auf einem Referenzstern 54 sind IRreflektierende Marker angebracht, die als Referenzmarkierungen für auf dem Patienten befindliche Marker genutzt werden können. Ein in die Liege integrierte Rahmen 56 ist wesentlich kürzer als die eigentliche Liege wodurch er nicht in Bereiche ragt, die strahlungsdurchlässig sein sollen.

Damit die Position des Patienten auf der Liege 50 während der Behandlung noch die gleiche ist wie die während der Aufnahme des Planungsdatensatzes darf sich der Patient zwischen diesen Zeitpunkten nicht bewegen. Die Liege wird deshalb vorzugsweise mit einem Patientenfixierungssystem kombiniert. Dazu sind unter anderen geeignet:
- Vakuumfolien mit entsprechenden Unterdruckpumpen
- Elastische Bänder oder Folien, die über den Patienten gespannt werden
- Speziell an die Form des Patienten angepasste Vakuumkissen
- Speziell an die Form des Patienten angepasste thermoplastische Körpermasken
- Formkissen oder -blöcke, die an die Form des Patienten angepasst werden

Der Stern 54 mit den Referenzmarkern ist fest mit der Liege 50 verbunden. Durch die Atmung des Patienten ändert sich der Abstand zwischen den Referenzmarkern und eventuellen Markern auf dem Thorax des Patienten. Dieser Abstand ist dazu geeignet die Atmung des Patienten zu beschreiben. Die Korrelation zwischen diesem Abstandswert und der Position interner Organe des Patienten bleibt erhalten, wenn die Liege 50 transportiert wird, da sowohl die Referenzmarker als auch der Patient fest mit der Liege verbunden sind. Ein (nicht dargestelltes) Trackingsystem am Planungsort (zum Beispiel CT) und am Behandlungsort (LINAC) unterstützt zum Beispiel eine atmungsgesteuerte Planung und Bestrahlung (getriggerte CT-Aufnahmen und getriggerte Bestrahlung), wobei dies erst möglich wird, weil die oben genannte Korrelation erhalten bleibt. Ein gewisser Toleranzbereich ist dabei einzuhalten. Die automatische Positionierung der Liege 50 am LINAC (computergestützt über das Trackingsystem und die Marker am Stern 54 an der Liege) erfolgt ebenfalls erst bei Erreichen des definierten Verhältnisses. Als Erweiterung ist es denkbar, an mehreren definierten Patienten-Fix-Marker Zuständen verschiedene CT-Sätze des gleichen Gebiets aufzunehmen, um mehrere "Anfangspunkte" schon beim CT bereitzustellen. Ein Arzt kann dann die Bewegung des Zielpunkts analysieren und ein "Beam-On-Range" bei der Bestrahlung (erlaubte Patienten-Fix-Marker-Beziehung für "Beam-On") kann individuell definiert werden. Wenn hier von CT-Planung die Rede ist, so soll dieses Verfahren nur als ein Beispiel eines bildgebenden Verfahrens stehen. Grundsätzlich ist die Verwendung anderer Verfahren möglich, zum Beispiel MR, SPECT, PET.

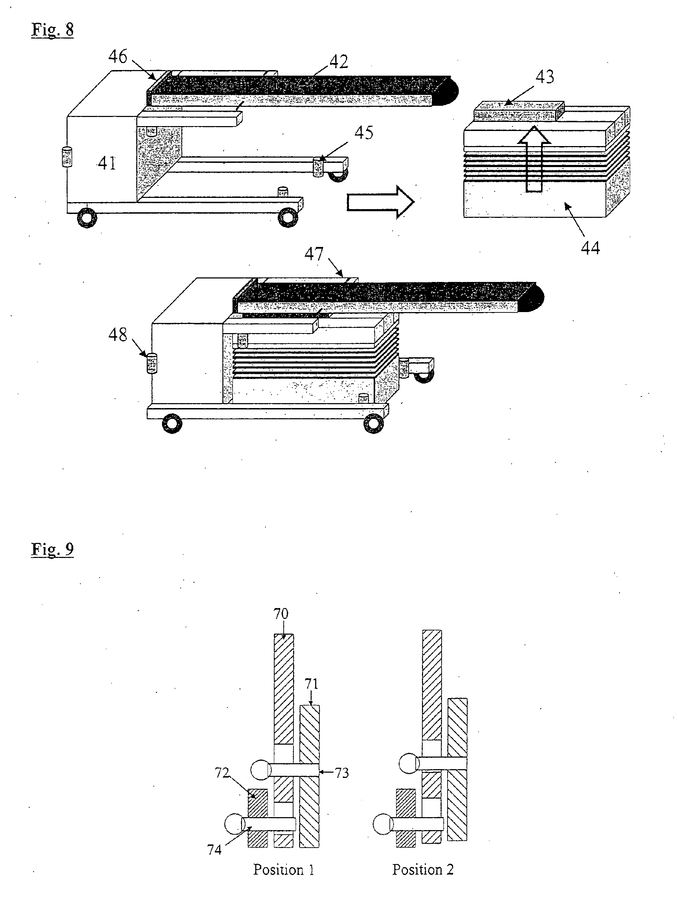

Um die Vorteile einer transportablen Patientenliege gut nutzen zu können, ist es vorteilhaft eine Vorrichtung zu haben, die es erlaubt, die Liege samt Patient vom bildgebenden System zum Behandlungsgerät transportieren zu können. Die Figur 8 zeigt eine Ausführungsform eines Transportsystems für eine Patientenliege 42. Ein Transportwagen 41 hält die Liege 42 in in etwa horizontaler Lage. Wird der Wagen 41 über den Tisch 44 des Behandlungsgerätes oder des bildgebenden Systems gefahren, so kann die Liege 42 auf die Verstelleinrichtung 43 übergeben werden. Der Wagen 41 ist mit seitlichen Rollen 45 an der Innenseite der zwei unteren Träger bestückt, so dass der Wagen 41 sich selbsttätig gegenüber dem Tisch 44 zentriert. Der Wagen 41 ist mit seitlichen Rollen 48 am liegenabgewandten Ende bestückt, sodass es möglich ist mit dem Wagen 41 Türen aufzuschieben.

Die Figur 9 zeigt eine mögliche Ausführungsform des Mechanismus zum Ein- und Ausklinken der Liege auf der Verstelleinrichtung oder direkt auf der Couch. Der wesentliche Vorteil dieses Mechanismus liegt darin, dass keine nennenswerte Handkraft aufgebracht werden muss, da die notwendige Kraft vom Tisch aufgebracht wird. Sowohl die Tische von Linearbeschleunigem als auch die von MR- und CT-Systemen erlauben es die Höhe des Tisches zu verstellen. Eine fest mit der Patientenliege verbundene Endplatte 70 (Bezugszeichen 46 in Figur 8) wird durch das vom Gewicht des überhängenden Patienten stammende Drehmoment nach oben gezogen. Ein Bolzen 73, der die Endplatte 70 mit dem Verstellmechanismus oder der Couch 71 verbindet, ist somit konstant mit einer Querkraft belastet. Der Bolzen 73 ist vorzugsweise mit Hinterschnitt gestaltet (in Figur 9 nicht sichtbar), so dass er nicht herausgezogen werden kann, wenn er mit Kraft belastet ist. Ein zweiter Bolzen 74 wird durch die mit dem Transportmittel für die Liege verbundenen Platte 74 in ein Langloch in die Endplatte 70 geführt. Solange der Bolzen 73 belastet ist, ist der Bolzen 74 unbelastet, so dass dieser ohne nennenswerte Kraft umgesteckt werden kann. Die Position 1 zeigt den Fall, bei dem die Liege mit dem Verstellmechanismus verbunden ist (Bolzen 73 trägt die Kraft).

Wird jetzt das Transportsystem an die Liege herangefahren, so können durch ein Herauffahren des Tisches die Liege und die Verstelleinrichtung und damit die Platten 70 und 71 nach oben gefahren werden, bis der Bolzen 74 am unteren Ende des Langloches anschlägt und von da an den Kraftfluss übernimmt. Wird der Tisch noch einige Millimeter weiter nach oben gefahren, so wird der Bolzen 73 ganz entlastet und kann von Hand herausgezogen werden. In umgekehrter Reihenfolge erfolgt die Abtrennung der Liege durch den Klinkmechanismus.

## Patentansprüche

1. Vorrichtung zur Patientenpositionierung im Rahmen der Behandlungsplanung mit Bilderfassung und/oder der Durchführung einer Bestrahlungsbehandlung, mit einer Patientenliege (3, 42, 50), auf der ein Patient gelagert wird, **dadurch gekennzeichnet, dass** Mittel vorgesehen sind, mit denen dieselbe Patientenliege (3, 42, 50) sowohl an einer Planungscouch (8) bei der Bilderfassung im Rahmen der Behandlungsplanung als auch an einem Bestrahlungstisch (1, 44) angebracht werden kann, und dadurch, dass an der Patientenliege ein Referenzmittel (54) vorgesehen ist, mit dem die Lage des immobilisierten Patienten bzw. markierter Punkte auf der Körperoberfläche des Patienten, gegenüber der Patientenliege (3, 42, 50) definiert bestimmbar ist.

2. Vorrichtung nach Anspruch 1, **dadurch gekennzeichnet, dass** sie ein Trackingsystem aufweist, welches sowohl die Positionen von auf dem Patienten angebrachten Markierungen als auch die des Referenzmittels, insbesondere eines mit einer Markeranordnung bestückten Referenzsterns (54), an der Liege (3, 42, 50) erfassen kann.

3. Vorrichtung nach Anspruch 1 oder 2, **dadurch gekennzeichnet, dass** sie eines oder mehrere der folgenden Immobilisierungsmittel zur Immobilisierung des Patienten auf der Patientenliege (3, 42, 50) umfasst:
- Vakuumfolien mit entsprechenden Unterdruckpumpen
- Elastische Bänder oder Folien, die über den Patienten gespannt werden
- Speziell an die Form des Patienten angepasste Vakuumkissen
- Speziell an die Form des Patienten angepasste thermoplastische Körpermasken
- Formkissen oder -blöcke, die an die Form des Patienten angepasst werden

4. Vorrichtung zur Patientenpositionierung im Rahmen der Behandlungsplanung mit Bilderfassung und/oder der Durchführung einer Bestrahlungsbehandlung, mit einer Patientenliege (3, 42, 50), auf der ein Patient gelagert wird, insbesondere nach einem der Ansprüche 1 bis 3, **gekennzeichnet durch** eine Verstelleinrichtung (6, 52), mit der die Patientenliege (3, 42, 50) um mindestens zwei Achsen gedreht werden kann und die zwischen der Patientenliege (3, 42, 50) und einer Stützeinrichtung für die Patientenliege (3, 42, 50), insbesondere einer Planungscouch (8) bzw. einem Behandlungstisch (1) angeordnet ist.

5. Vorrichtung nach Anspruch 4, **dadurch gekennzeichnet, dass** die Verstelleinrichtung (6, 52) ein unabhängiges Drehen der Patientenliege (3, 42, 50) um mindestens eine Achse quer und/oder eine Achse längs zur Liege (3, 42, 50) ermöglicht.

6. Vorrichtung nach Anspruch 4 oder 5, **dadurch gekennzeichnet, dass** die Verstelleinrichtung (6, 52) aus einer Grund- und einer Deckplatte (10, 12) besteht, die über eine Dreipunkt- oder Vierpunktlagerung miteinander beweglich verbunden sind.

7. Vorrichtung nach Anspruch 5 oder 6, **dadurch gekennzeichnet, dass** die Verstelleinrichtung (6, 52) mindestens zwei Auflager (16, 17) aufweist, die als höhenverstellbare, pneumatische, hydraulische, piezoelektrische oder elektromechanische Auflager, direkt oder mittels eines Hebelsystems verstellbar, ausgebildet sind.

8. Vorrichtung nach Anspruch 6 oder 7, **dadurch gekennzeichnet, dass** die Verstelleinrichtung (6, 52) eine vordere, kopfseitige Auflagerung und eine hintere, fußseitige Auflagerung aufweist, wobei mindestens eine der Auflagerungen als Kugelgelenk- oder Kardanlager ausgebildet ist.

9. Vorrichtung nach Anspruch 8, **dadurch gekennzeichnet, dass** die vordere oder die hintere Auflagerung Kniehebel mit beiseitigen Kugelgelenken aufweist.

10. Vorrichtung nach einem der Ansprüche 6 bis 9, **dadurch gekennzeichnet, dass** die Lagerung der Deckplatte (12) auf der Grundplatte (10) ein Gelenk (22, 23) mit zwei translatorischen und zwei rotatorischen Freiheitsgraden umfasst, welches ein seitliches Verschieben der Deckplatte (12) gegenüber der Grundplatte (10) verhindert.

11. Vorrichtung nach einem der Ansprüche 7 bis 10, **dadurch gekennzeichnet, dass** sie Verstellmittel aufweist, welche die Rotation der Liege (3, 42, 50) um ihre Längsachse durch gegenläufiges Verstellen von zwei kopf- oder fußseitigen Auflagern bewirken.

12. Vorrichtung nach einem der Ansprüche 7 bis 11, **dadurch gekennzeichnet, dass** sie Verstellmittel aufweist, welche die Rotation der Liege (3, 42, 50) um ihre Querachse durch gleichläufiges Verstellen eines oder zweier kopf- oder fußseitiger Auflager bewirken.

13. Vorrichtung nach einem der Ansprüche 7 bis 12, **dadurch gekennzeichnet, dass** sie Verstellmittel aufweist, welche die Rotation der Liege (3, 42, 50) um ihre Querachse durch gleichzeitiges Verstellen mindestens eines kopfseitigen und mindestens eines fußseitigen Auflagers bewirken.

14. Vorrichtung nach einem der Ansprüche 7 bis 13, **dadurch gekennzeichnet, dass** sie Verstellmittel aufweist, welche die Rotation der Liege (3, 42, 50) um ihre Querachse durch gezieltes Einstellen der Verstell-Geschwindigkeit mindestens eines kopfseitigen und mindestens eines fußseitigen Auflagers bewirken, wodurch die Lage der scheinbaren Drehachse in eine gewollte Position gebracht werden kann.

15. Vorrichtung nach einem der Ansprüche 7 bis 14, **dadurch gekennzeichnet, dass** sie Verstellmittel aufweist, welche die Rotation der Liege (3, 42, 50) um ihre Längsachse durch gezieltes Einstellen der Verstell-Geschwindigkeit mindestens zweier Auflager bewirkt, wodurch die Lage der scheinbaren Drehachse in eine gewollte Position gebracht werden kann.

16. Vorrichtung zur Patientenpositionierung im Rahmen der Behandlungsplanung mit Bilderfassung und/oder der Durchführung einer Bestrahlungsbehandlung, mit einer Patientenliege (3, 42, 50), auf der ein Patient gelagert wird, insbesondere nach einem der Ansprüche 1 bis 15, **dadurch gekennzeichnet, dass** sie eine Transportvorrichtung, insbesondere einen Transportwagen (41) zum Transportieren der Liege (3, 42, 50) und des darauf liegenden Patienten umfasst.

17. Vorrichtung nach Anspruch 16, **dadurch gekennzeichnet, dass** zur Verbindung von Patientenliege (3, 42, 50) und Transportwagen (41) zwei Einklinkmechanismen vorgesehen sind, zum Einhängen der Patientenliege (3, 42, 50) an einem Tisch (1) eines Bestrahlungssystems an und einer Couch (8) eines bildgebenden Planungssystems (2).

18. Vorrichtung nach einem der Ansprüche 16 oder 17, **dadurch gekennzeichnet, dass** das Ein- und Aushängen der Liege (3, 42, 50) beim Tisch (1) des Bestrahlungsgeräts oder bei der Couch (8) des Planungssystems (2) über die Höhenverstellung des Tisches (1) bzw. der Couch (8) erfolgt.

19. Vorrichtung nach einem der Ansprüche 16 bis 18, **dadurch gekennzeichnet, dass** das Ein- und Aushängen der Liege (3, 42, 50) beim Tisch (1) des Bestrahlungsgeräts oder bei der Couch (8) des Planungssystems (2) über die Höhenverstellung der Transportvorrichtung erfolgt.

20. Verfahren zur Behandlungsplanung mit Bilderfassung und/oder Durchführung einer Bestrahlungsbehandlung, bei dem ein Patient auf einer Patientenliege (3, 42, 50) gelagert wird, bei dem dieselbe Patientenliege (3, 42, 50) sowohl an einer Planungscouch (8) bei der Bilderfassung im Rahmen der Behandlungsplanung als auch an einem Bestrahlungstisch (1, 44) genutzt wird, und bei dem die Lage des immobilisierten Patienten bzw. markierter Punkte auf der Körperoberfläche des Patienten, gegenüber der Patientenliege (3, 42, 50) über ein Referenzmittel (54) bestimmt wird, das fest an der Patientenliege vorgesehen ist.

21. Verfahren nach Anspruch 20, bei dem die Liege (3, 42, 50), auf welcher der Patient liegt, von der Planungscouch (8) abgenommen, zum Behandlungsgerät transportiert und dort wieder befestigt wird.

22. Verfahren nach Anspruch 20 oder 21, bei dem ein Trackingsystem am bilderfassenden Gerät sowohl die Positionen von auf dem Patienten angebrachten Markierungen als auch die der Referenzmarkierungen auf der Liege erfasst, bzw. nach diesen eingestellt wird.

23. Verfahren nach einem der Ansprüche 20 bis 22, bei dem der Patient auf der Liege (3, 42, 50) so fixiert wird, dass er sich weder relativ zur Liege verschieben kann, noch die Lage seiner Körperteile zueinander ändern kann.

24. Verfahren nach einem der Ansprüche 20 bis 24, zur atemsynchronisierten Bestrahlung, bei dem der fixierte Patient mit externen Markierungen bestückt wird, wobei anhand der Position dieser Markierungen relativ zu dem Referenzmittel (54) verifiziert wird, dass der Patient die Atmung während der Aufnahme der Planungsdaten angehalten hat.

25. Verfahren nach einem Ansprüche 20 bis 24, bei dem die relative Position von Patientenmarkierungen zu dem Referenzmittel (54) ermittelt und bei Übereinstimmung mit toleranzbehafteten Vorgaben eine Bildaufnahme gestartet oder gestoppt wird, wenn der Patient die Luft anhält, die Atmung wieder aufnimmt, eine bestimmte Lungenfüllung erreicht hat oder eine bestimmte Lungenfüllung in einer vorgegebenen Atemphase, zum Beispiel während des Einatmens oder Ausatmens durchschreitet.

26. Verfahren nach einem der Ansprüche 20 bis 25, bei dem der Patient nur zu Zeitpunkten bestrahlt wird, an denen die relative Position von Patientenmarkierungen zur Position des fest auf der Liege (3, 42, 54) angebrachten Referenzmittels (54) in einem definierten Toleranzbereich um die relative Position liegt, die während der Aufnahme eines Planungsdatensatzes vorlag.
